Europäisches Patentamt

European Patent Office

Office européen des brevets

(19)

(11) **EP 1 460 559 A1**

(12) **EUROPEAN PATENT APPLICATION**
published in accordance with Art. 158(3) EPC

(43) Date of publication:
**22.09.2004 Bulletin 2004/39**

(51) Int Cl.⁷: **G06F 17/30**, G06F 19/00,
C07K 1/00

(21) Application number: **02792060.2**

(22) Date of filing: **27.12.2002**

(86) International application number:
**PCT/JP2002/013832**

(87) International publication number:
**WO 2003/056461 (10.07.2003 Gazette 2003/28)**

(84) Designated Contracting States:
**AT BE BG CH CY CZ DE DK EE ES FI FR GB GR
IE IT LI LU MC NL PT SE SI SK TR**

(30) Priority: **27.12.2001 JP 2001398569**

(71) Applicant: **Celestar Lexico-Sciences, Inc.
Chiba-shi, Chiba 261-8501 (JP)**

(72) Inventor: **SAITO, Seiji,c/o Celestar
Lexico-Sciences, Inc.
Chiba-shi, Chiba 261-8501 (JP)**

(74) Representative: **Grill, Matthias, Dipl.-Ing.
Patentanwälte
Tiedtke-Bühling-Kinne & Partner
Bavariaring 4
80336 München (DE)**

(54) **PROTEIN STRUCTURE PREDICTING DEVICE, PROTEIN STRUCTURE PREDICTING METHOD, PROGRAM, AND RECORDING MEDIUM**

(57)     According to the present invention, which structure cluster a sequence present around a sequence A and resembling the sequence A belongs to in a structure space (which structure cluster a sequence belongs to when the sequence resembles in what way) is determined by calculation, and a virtual cluster is created around the sequence. When an unknown structure sequence fragment X is given, information on whether the fragment resembles sequence A or C is collected, virtual clusters are combined depending on the information, and a which structure cluster the sequence belongs to is predicted finally.

## FIG.3

(BASIC PRINCIPLE OF THE PRESENT INVENTION)

**Description**

TECHNICAL FIELD

The present invention relates to a protein structure prediction device, a protein structure prediction method, a program, and a recording medium. More specifically, the present invention relates to a protein structure prediction device, a protein structure prediction method, a program, and a recording medium for predicting a protein structure by a correlation between a sequence and a structure.

BACKGROUND ART

It is said that a protein structure is determined solely by sequence information. Namely, there is some correlation between a sequence space and a structure space. Here, if we can compare the size of the sequence space with that of the structure space (native structure space), the size of the sequence space may be larger. This is because even if a sequence changes a little, the structure of the sequence does not appear to greatly change evolutionally. In other words, the structure is more evolutionally conservative than the sequence.

Further, it becomes clear from the recent analysis of evolutionally similar proteins that proteins having similar sequences are similar in overall structure. Based on the fact that the overall structure consists of a combination of parts, it is possible to assume that this rule of thumb that may stand for the overall protein structure also stand for a region taken out of parts of a protein to some extent.

There actually exist proteins which have a correlation between a local sequence and a local structure, i.e., for which a similar local protein sequence provides a similar local structure. In recent studies, the overall structure is tried so as to be assembled out of local sequences using the correlation between the local sequence and the local structure.

In studies disclosed by "Assembly of Protein Tertiary Structures from Fragments with Similar Local Sequences using Simulated Annealing and Bayesian Scoring Functions", by Kim T. Simons, et al., J. Mol. Biol. (1997) 268, P. 209 to P. 225 (hereinafter, "Literature 1") and "Prediction of Local Structure in Proteins Using a Library of Sequence-Structure Motifs" by Christopher Bystroff, et al., J. Mol. Biol. (1998) 281, P. 565 to P. 577 (hereinafter, "Literature 2"), for example, by clustering a structure corresponding to a local sequence, a wide structure (folding) space can be narrowed and time for calculating a folding simulation can be reduced.

The Literature 1 discloses that the structure space is reduced since the local structure is restricted to a specific offset structure by the local sequence, the structure is similar to the structure of a protein having a similar sequence, and that a sequence profile is obtained by multiple alignment to thereby obtain a proximity of each sequence to a query sequence.

The Literature 2 discloses that if a fragment structure correlates to a sequence, a limited number of structure candidates can be determined from a sequence fragment tendency, the structures are clustered using two structure indexes, each sequence is calculated using a distance of a frequency profile, and that similar structures are searched from those similar in sequence and clustered, thereby actually creating sequence and structure fragment clusters.

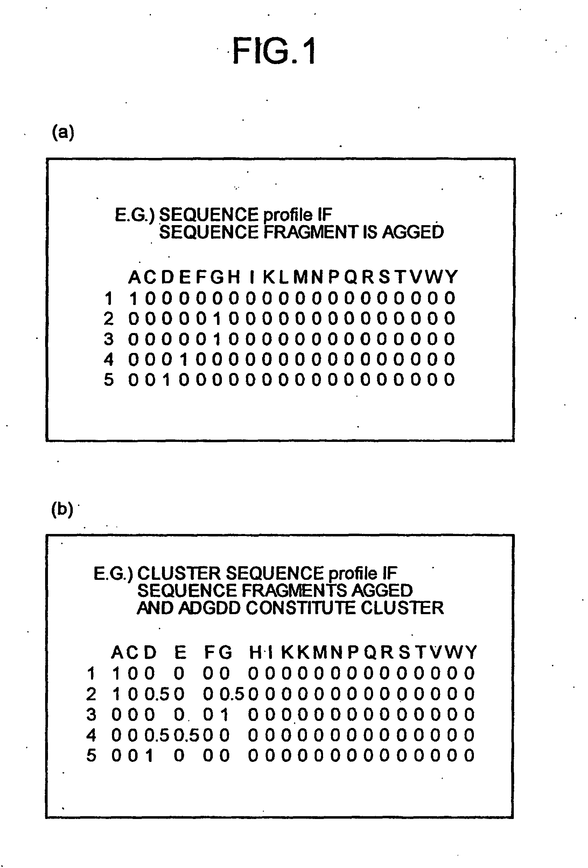

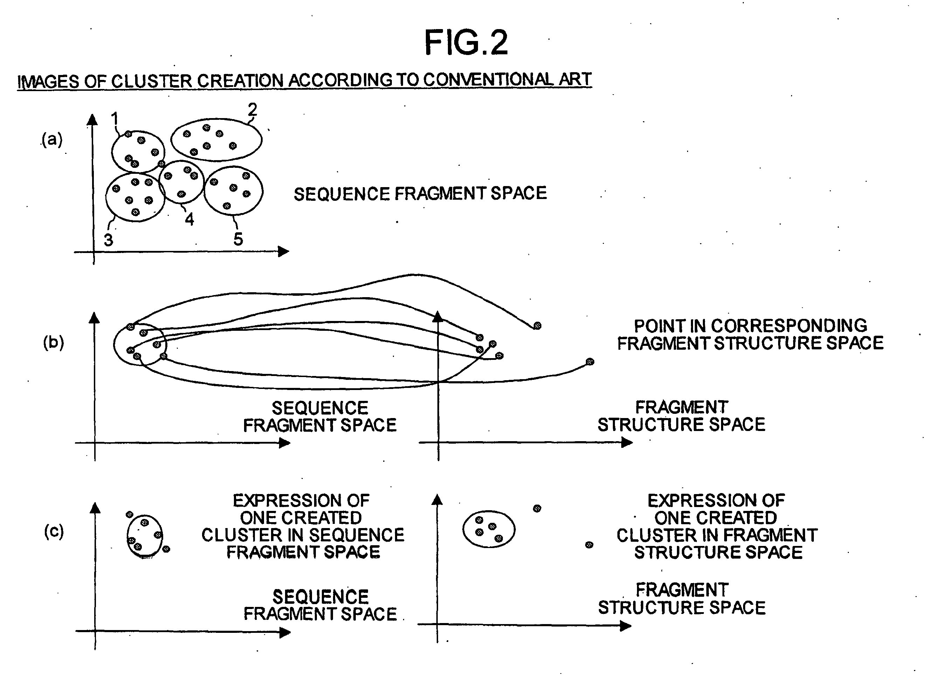

A conventional structure cluster creation process will be explained with reference to Figs. 1 and 2. Fig. 1 illustrates one example in which a sequence is expressed by a profile according to the conventional art. Fig. 2 illustrates images of structure cluster creation according to the conventional art.

A sequence is expressed by the profile first. As shown in Fig. 1 (a), a profile is created by setting "1" to amino acids corresponding to a sequence (AGGED). In addition, if sequences (AGGED) and (ADGDD), for example, constitute one cluster, a profile of this cluster is created as shown in Fig. 1(b). Namely, a frequency of an amino acid present at a certain position is set in relation to a sequence that belongs to the cluster, thereby creating the profile. By comparing the sequence and the cluster based on their respective profiles, the similarity between one sequence and the cluster can be calculated.

Sequences are clustered in the sequence space so that those similar in sequence profile belong to the same clusters (1 to 5 in Fig. 2(a)). That is, similarities of sequence profiles are calculated and similarities of all the sequences are calculated, thereby creating equidistant clusters.

Thereafter, a correlation is determined as to which point each of the sequences corresponds to in the structure space (the correlation between each sequence and a point in the structure for the cluster 1 is determined in the Fig. 2(b)), and the sequences having high correlations between the sequence and the structure are clustered (Fig. 2(c)). That is, the sequences which are included in each cluster in the sequence space shown in Fig. 2(b) and the points corresponding to which in the structure space are close (the sequences similar in structure) are extracted while those which are not similar in structure are discarded. The processing is repeated using clusters thus created and the discarded sequences, thereby creating structure clusters.

In these conventional methods, the static clusters based on the sequence profiles can only have equidistant correlations. However, these correlations appear to form a complex manifold. Therefore, these conventional methods have a disadvantage in that it cannot represent these complex correlations.

Further, although it is true that the overall structure is formed out of local structures, there should be sequences having a locally high correlation, those having a locally low correlation, those correlations of which cannot be determined, and the like. Therefore, the conventional methods have a disadvantage in that quantification of these sequences is insufficient.

It is, therefore, an object of the present invention to provide a protein structure prediction device, a protein structure prediction method, a program, and a recording medium capable of calculating a correlation of a local structure from a local sequence so as to be able to express a complex manifold of the correlation and even a certainty factor of the correlation.

DISCLOSURE OF THE INVENTION

A protein structure prediction device according to one aspect of the present invention includes a fragment structure cluster creation unit that based on sequence information and three-dimensional structure information on a protein, creates a plurality of sequence fragments obtained by dividing the sequence information at intervals of a predetermined length and a plurality of fragment structures corresponding to the respective sequence fragments, and that creates a plurality of fragment structure clusters based on similarities of the fragment structures; a sequence fragment similarity search unit that performs sequence similarity search for similarities between the sequence fragments and the sequence fragments present around the sequence fragments in a sequence space, respectively; a certainty factor matrix creation unit that creates a certainty factor matrix which represents a certainty factor in a form of a matrix of the sequence fragments and the structure clusters, the certainty factor being a probability that the similar sequence belongs to a fragment structure cluster out of the fragment structure clusters; a query sequence input unit that allows a user to input a query sequence; a query sequence fragment creation unit that divides the query sequence input by the query sequence input unit at intervals of a predetermined length to create query sequence fragments; a query sequence fragment similarity search unit that performs sequence similarity search for similarities between each of the query sequence fragments created by the query sequence fragment creation unit and each of the sequence fragments; a fragment structure probability calculation unit that calculates a probability that each of the query sequence fragments belongs to each of the fragment structure clusters based on the certainty factor matrix created by the certainty factor matrix creation unit and a search result of the query sequence fragment similarity search unit; and a sequence fragment structure prediction unit that predicts a fragment structure of the query sequence based on the probability calculated by the fragment structure probability calculation unit.

According to this device, sequence fragments obtained by dividing sequence information at intervals of a predetermined length and fragment structures corresponding to the sequence fragments are created based on the sequence information and three-dimensional structure information on a protein, fragment structure clusters are created based on similarities of the fragment structures, sequence similarity searches are conducted for similarities between the sequence fragments and the sequence fragments present around the sequence fragments in a sequence space, respectively, and a certainty factor matrix representing a certainty factor that is a probability that a sequence resembling each of the sequence fragments belongs to each of the fragment structure clusters in the form of a matrix of the sequence fragments and the structure clusters is created. A user is allowed to input a query matrix, the input query sequence is divided at intervals of a predetermined length to thereby create query sequence fragments, sequence similarity searches are conducted for similarities between the created query sequence fragments and the sequence fragment, respectively, the probability that each of the query sequence fragments belongs to each of the fragment structure clusters is calculated based on the created certainty factor matrix and search results, and the fragment structure of the query sequence is predicted based on the calculated probability. Therefore, it is possible to calculate a correlation of a local structure from a local sequence so as to be able to express a complex manifold of the correlation and predict the partial structure. Namely, the present invention can provide the protein structure prediction device capable of giving and holding probabilities (certainty factors) of a plurality of structure candidates according to their degrees of correlations when calculating the structure (using a certainty factor function as a probability of structure change).

Further, the technique that the protein structure is assumed as a block of local structures each having a high correlation is conventionally known. However, the protein structure prediction device can create clusters of local structures first, consider a complex form of a structure sequence correlation manifold, and dynamically create sequence correlation clusters after the query sequence is given.

In addition, the protein structure prediction device can create many structure clusters from different viewpoints (for example, lengths of sequence fragments, resolutions of fragment structures, magnitudes of structure clusters, and degrees of correlations) and calculate the structure by integrating structure prediction results from the respective datasets.

The protein structure prediction device according to another aspect of the present invention further includes a similarity matrix creation unit that creates a similarity matrix which represents a search result of the sequence fragment similarity search unit in a form of a matrix of the sequence fragments; and a structure cluster information matrix creation unit that creates a structure cluster information matrix which represents structure cluster information in a form of a matrix of the sequence fragments and the structure clusters, the structure cluster information indicating which of the fragment structure clusters each of the sequence fragments belongs to, wherein the certainty factor matrix creation unit creates the certainty factor matrix based on the similarity matrix and the structure cluster information matrix.

This aspect indicates an example of the certainty factor matrix in more detail. According to the protein structure prediction device, a similarity matrix creation unit which creates a similarity matrix representing the results of the similarity searches conducted by the sequence fragment similarity search unit for the sequence fragments in the form of a matrix of the sequence fragments is provided, a cluster information matrix representing structure cluster information that indicates which of the fragment structure clusters each of the sequence fragments belongs to in the form of a matrix of the sequence fragments and the structure clusters is created, and the certainty factor matrix is created based on the similarity matrix and the structure cluster information matrix thus created. Therefore, the protein structure prediction device is capable of easily, finely calculating certainty factors based on the similarity search results using a matrix operation technique.

The protein structure prediction device according to still another aspect of the present invention further includes an overall structure optimization unit that performs predetermined optimization to an initial overall structure determined by a fragment structure, out of the fragment structures, having a highest certainty factor.

According to the protein structure prediction device, predetermined optimization is conducted to an initial overall structure determined by the fragment structure having the highest certainty factor. Therefore, the sequence can be divided to various possible sequence fragments and their optimum prediction results can be integrated when creating the initial structure. In addition, the protein structure prediction device is capable of further improving accuracy for the overall structure prediction by further optimizing the initial structure.

Moreover, a protein structure prediction method according to still another aspect of the present invention includes a fragment structure cluster creation step of creating, based on sequence information and three-dimensional structure information on a protein, a plurality of sequence fragments obtained by dividing the sequence information at intervals of a predetermined length and a plurality of fragment structures corresponding to the respective sequence fragments, and of creating a plurality of fragment structure clusters based on similarities of the fragment structures; a sequence fragment similarity search step of performing sequence similarity search for similarities between the sequence fragments and the sequence fragments present around the sequence fragments in a sequence space, respectively; a certainty factor matrix creation step of creating a certainty factor matrix which represents a certainty factor in a form of a matrix of the sequence fragments and the structure clusters, the certainty factor being a probability that the similar sequence belongs to a fragment structure cluster out of the fragment structure clusters; a query sequence input step of allowing a user to input a query sequence; a query sequence fragment creation step of dividing the query sequence at intervals of a predetermined length to create query sequence fragments; a query sequence fragment similarity search step of performing sequence similarity search for similarities between each of the query sequence fragments created by the query sequence fragment creation step and each of the sequence fragments; a fragment structure probability calculation step of calculating a probability that each of the query sequence fragments belongs to each of the fragment structure clusters based on the certainty factor matrix created by the certainty factor matrix creation step and a search result of the performing sequence similarity search; and a sequence fragment structure prediction step of predicting a fragment structure of the query sequence based on the probability calculated by the fragment structure probability calculation step.

According to this method, sequence fragments obtained by dividing sequence information at intervals of a predetermined length and fragment structures corresponding to the sequence fragments are created based on the sequence information and three-dimensional structure information on a protein, fragment structure clusters are created based on similarities of the fragment structures, sequence similarity searches are conducted for similarities between the sequence fragments and the sequence fragments present around the sequence fragments in a sequence space, respectively, and a certainty factor matrix representing a certainty factor that is a probability that a sequence resembling each of the sequence fragments belongs to each of the fragment structure clusters in the form of a matrix of the sequence fragments and the structure clusters is created. A user is allowed to input a query matrix, the input query sequence is divided at intervals of a predetermined length to thereby create query sequence fragments, sequence similarity searches are conducted for similarities between the created query sequence fragments and the sequence fragment, respectively, the probability that each of the query sequence fragments belongs to each of the fragment structure clusters is calculated based on the created certainty factor matrix and search results, and the fragment structure of the query sequence is predicted based on the calculated probability. Therefore, it is possible to calculate a correlation of a local structure from a local sequence so as to be able to express a complex manifold of the correlation and predict the partial structure. Namely, the present invention can provide the protein structure prediction method capable of giving and holding probabilities (certainty factors) of a plurality of structure candidates according to their degrees of correlations when calculating the structure (using a certainty factor function as a probability of structure change).

Further, the technique that the protein structure is assumed as a block of local structures each having a high correlation is conventionally known. However, the protein structure prediction method can create clusters of local structures first, consider a complex form of a structure sequence correlation manifold, and dynamically create sequence correlation clusters after the query sequence is given.

In addition, the protein structure prediction method can create many structure clusters from different viewpoints (for example, lengths of sequence fragments, resolutions of fragment structures, magnitudes of structure clusters, and degrees of correlations) and calculate the structure by integrating structure prediction results from the respective datasets.

The protein structure prediction method according to still another aspect of the present invention further includes a similarity matrix creation step of creating a similarity matrix which represents a search result of the sequence fragment similarity search unit in a form of a matrix of the sequence fragments; and a structure cluster information matrix creation step of creating a structure cluster information matrix which represents structure cluster information in a form of a matrix of the sequence fragments and the structure clusters, the structure cluster information indicating which of the fragment structure clusters each of the sequence fragments belongs to, wherein the certainty factor matrix creation step includes creating the certainty factor matrix based on the similarity matrix created by the similarity matrix creation step and the structure cluster information matrix created by the similarity matrix creation step.

This aspect indicates an example of the certainty factor matrix in more detail. According to the protein structure prediction method, a similarity matrix creation unit which creates a similarity matrix representing the results of the similarity searches conducted by the sequence fragment similarity search unit for the sequence fragments in the form of a matrix of the sequence fragments is provided, a cluster information matrix representing structure cluster information that indicates which of the fragment structure clusters each of the sequence fragments belongs to in the form of a matrix of the sequence fragments and the structure clusters is created, and the certainty factor matrix is created based on the similarity matrix and the structure cluster information matrix thus created. Therefore, the protein structure prediction method is capable of easily, finely calculating certainty factors based on the similarity search results using a matrix operation technique.

The protein structure prediction method according to still another aspect of the present invention further includes an overall structure optimization step of performing predetermined optimization to an initial overall structure determined by a fragment structure, out of the fragment structures, having a highest certainty factor.

According to the protein structure prediction method, predetermined optimization is conducted to an initial overall structure determined by the fragment structure having the highest certainty factor. Therefore, the sequence can be divided to various possible sequence fragments and their optimum prediction results can be integrated when creating the initial structure. In addition, the protein structure prediction method is capable of further improving accuracy for the overall structure prediction by further optimizing the initial structure.

Moreover, a program according to still another aspect of the present invention allows a computer to execute a protein structure prediction method that includes a fragment structure cluster creation step of creating, based on sequence information and three-dimensional structure information on a protein, a plurality of sequence fragments obtained by dividing the sequence information at intervals of a predetermined length and a plurality of fragment structures corresponding to the respective sequence fragments, and of creating a plurality of fragment structure clusters based on similarities of the fragment structures; a sequence fragment similarity search step of performing sequence similarity search for similarities between the sequence fragments and the sequence fragments present around the sequence fragments in a sequence space, respectively; a certainty factor matrix creation step of creating a certainty factor matrix which represents a certainty factor in a form of a matrix of the sequence fragments and the structure clusters, the certainty factor being a probability that the similar sequence belongs to a fragment structure cluster out of the fragment structure clusters; a query sequence input step of allowing a user to input a query sequence; a query sequence fragment creation step of dividing the query sequence at intervals of a predetermined length to create query sequence fragments; a query sequence fragment similarity search step of performing sequence similarity search for similarities between each of the query sequence fragments created by the query sequence fragment creation step and each of the sequence fragments; a fragment structure probability calculation step of calculating a probability that each of the query sequence fragments belongs to each of the fragment structure clusters based on the certainty factor matrix created by the certainty factor matrix creation step and a search result of the performing sequence similarity search; and a sequence fragment structure prediction step of predicting a fragment structure of the query sequence based on the probability calculated by the fragment structure probability calculation step.

According to this program, sequence fragments obtained by dividing sequence information at intervals of a predetermined length and fragment structures corresponding to the sequence fragments are created based on the sequence information and three-dimensional structure information on a protein, fragment structure clusters are created based on similarities of the fragment structures, sequence similarity searches are conducted for similarities between the sequence fragments and the sequence fragments present around the sequence fragments in a sequence space, respectively, and a certainty factor matrix representing a certainty factor that is a probability that a sequence resembling each of the sequence fragments belongs to each of the fragment structure clusters in the form of a matrix of the sequence fragments and the structure clusters is created. A user is allowed to input a query matrix, the input query sequence is divided at intervals of a predetermined length to thereby create query sequence fragments, sequence similarity searches are conducted for similarities between the created query sequence fragments and the sequence fragment, respectively, the probability that each of the query sequence fragments belongs to each of the fragment structure clusters is calculated based on the created certainty factor matrix and search results, and the fragment structure of the query sequence is predicted based on the calculated probability. Therefore, it is possible to calculate a correlation of a local structure from a local sequence so as to be able to express a complex manifold of the correlation and predict the partial structure. Namely, the present invention can provide the program capable of giving and holding probabilities (certainty factors) of a plurality of structure candidates according to their degrees of correlations when calculating the structure (using a certainty factor function as a probability of structure change).

Further, the technique that the protein structure is assumed as a block of local structures each having a high correlation is conventionally known. However, the program can create clusters of local structures first, consider a complex form of a structure sequence correlation manifold, and dynamically create sequence correlation clusters after the query sequence is given.

In addition, the program can create many structure clusters from different viewpoints (for example, lengths of sequence fragments, resolutions of fragment structures, magnitudes of structure clusters, and degrees of correlations) and calculate the structure by integrating structure prediction results from the respective datasets.

In the program according to still another aspect of the present invention, the protein structure prediction method further includes a similarity matrix creation step of creating a similarity matrix which represents a search result of the sequence fragment similarity search unit in a form of a matrix of the sequence fragments; and a structure cluster information matrix creation step of creating a structure cluster information matrix which represents structure cluster information in a form of a matrix of the sequence fragments and the structure clusters, the structure cluster information indicating which of the fragment structure clusters each of the sequence fragments belongs to, wherein the certainty factor matrix creation step includes creating the certainty factor matrix based on the similarity matrix created by the similarity matrix creation step and the structure cluster information matrix created by the similarity matrix creation step.

This aspect indicates an example of the certainty factor matrix in more detail. According to the program, a similarity matrix creation unit which creates a similarity matrix representing the results of the similarity searches conducted by the sequence fragment similarity search unit for the sequence fragments in the form of a matrix of the sequence fragments is provided, a cluster information matrix representing structure cluster information that indicates which of the fragment structure clusters each of the sequence fragments belongs to in the form of a matrix of the sequence fragments and the structure clusters is created, and the certainty factor matrix is created based on the similarity matrix and the structure cluster information matrix thus created. Therefore, the program is capable of easily, finely calculating certainty factors based on the similarity search results using a matrix operation technique.

In the program according to still another aspect of the present invention, the protein structure prediction method further includes an overall structure optimization step of performing predetermined optimization to an initial overall structure determined by a fragment structure, out of the fragment structures, having a highest certainty factor.

According to the program, predetermined optimization is conducted to an initial overall structure determined by the fragment structure having the highest certainty factor. Therefore, the sequence can be divided to various possible sequence fragments and their optimum prediction results can be integrated when creating the initial structure. In addition, the protein structure prediction method is capable of further improving accuracy for the overall structure prediction by further optimizing the initial structure.

Furthermore, the present invention relates to a recording medium, wherein the recording medium has the above described program recorded therein.

In the recording medium, by making a computer read and executed the program recorded in the medium, the above-described program can be realized by using a computer, and similar effects to each program can be obtained.

BRIEF DESCRIPTION OF THE DRAWINGS

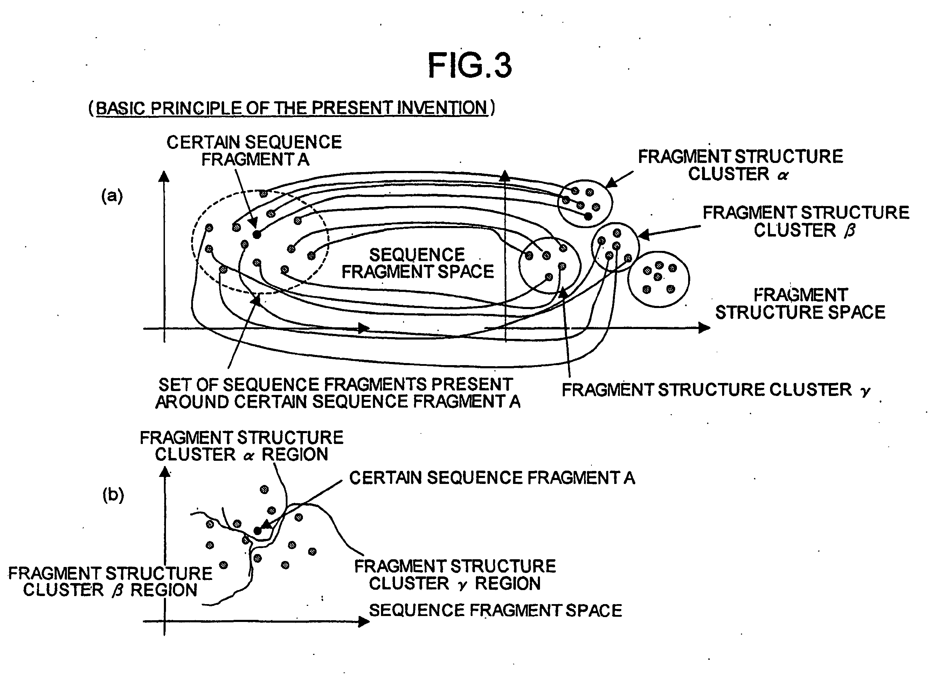

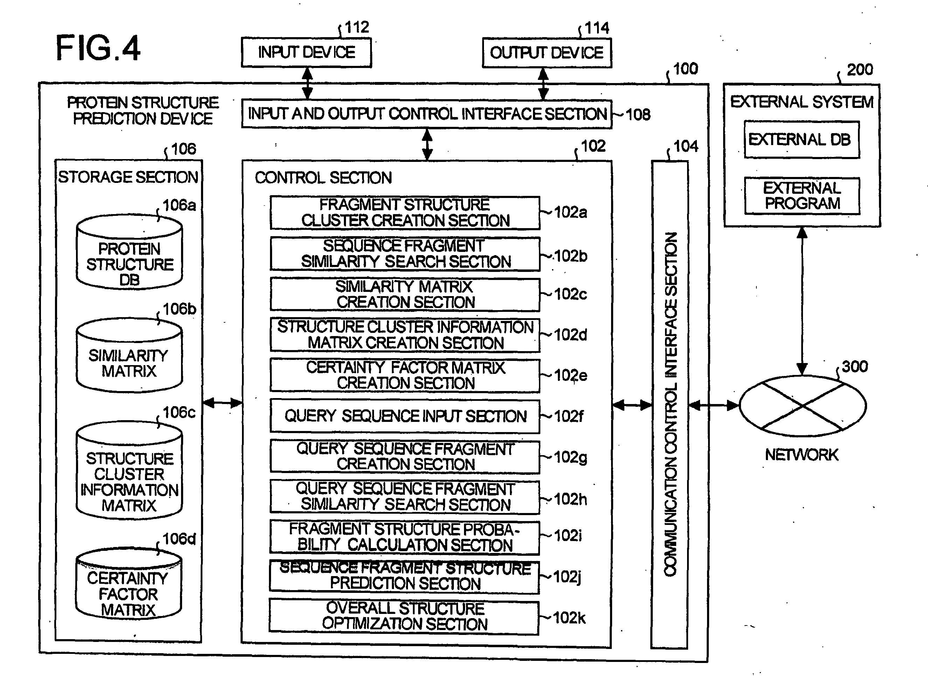

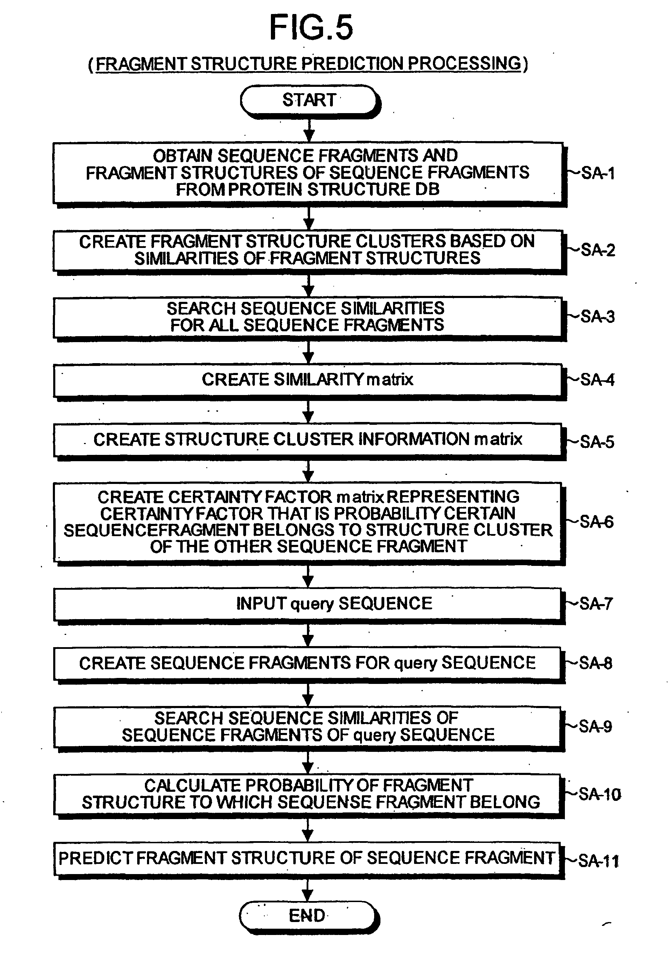

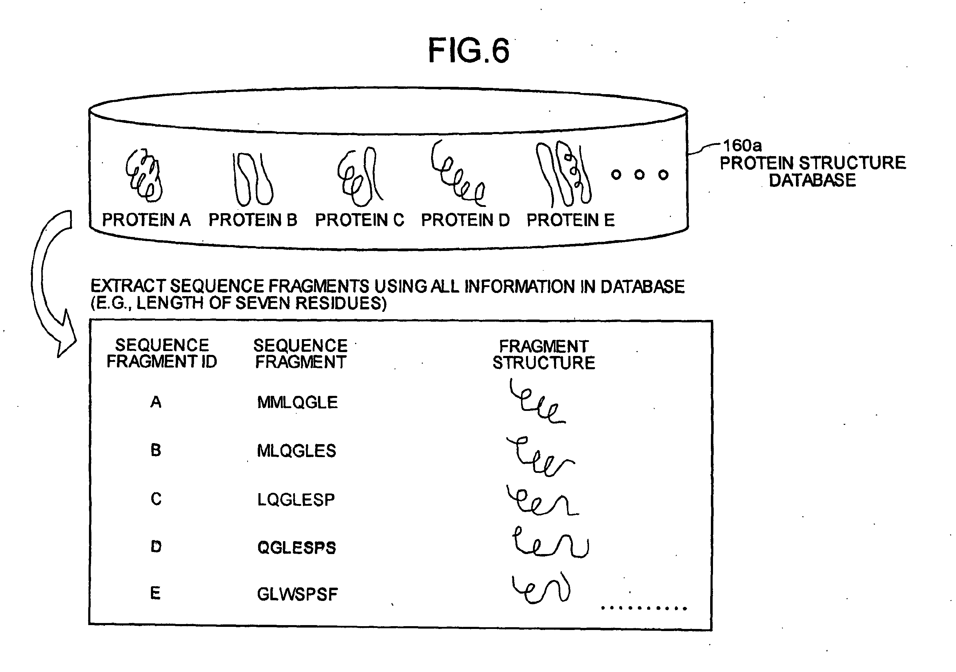

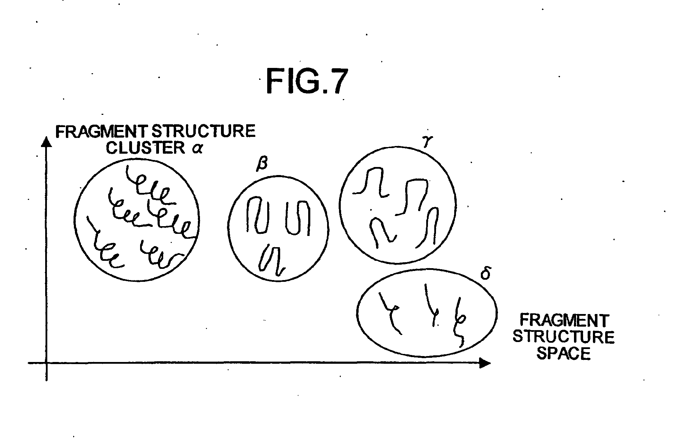

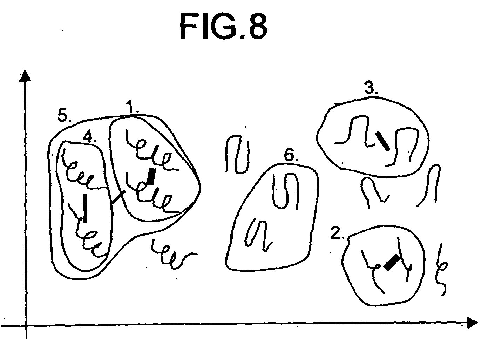

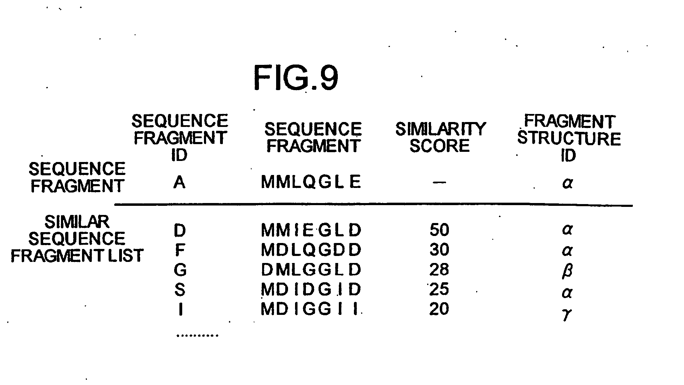

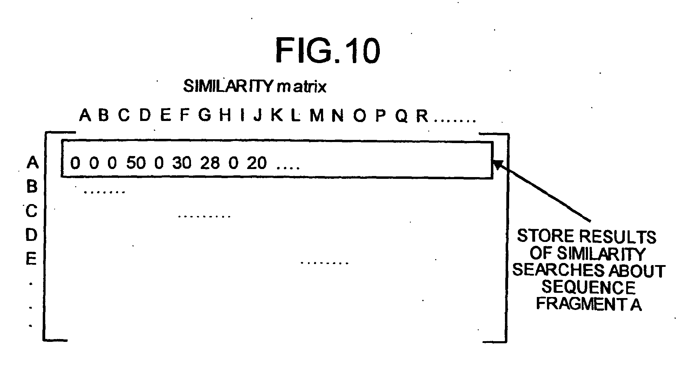

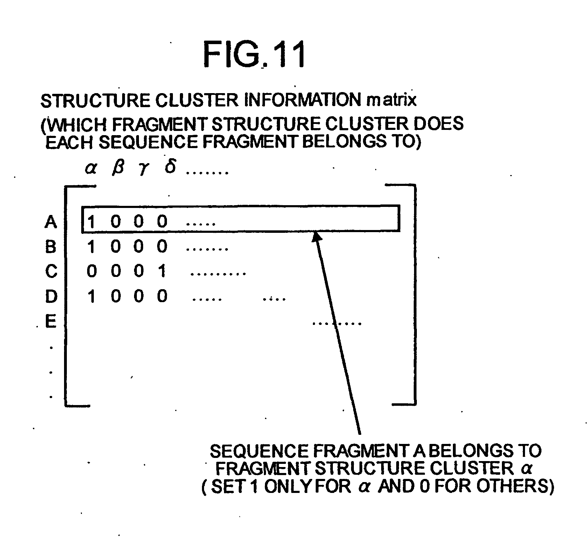

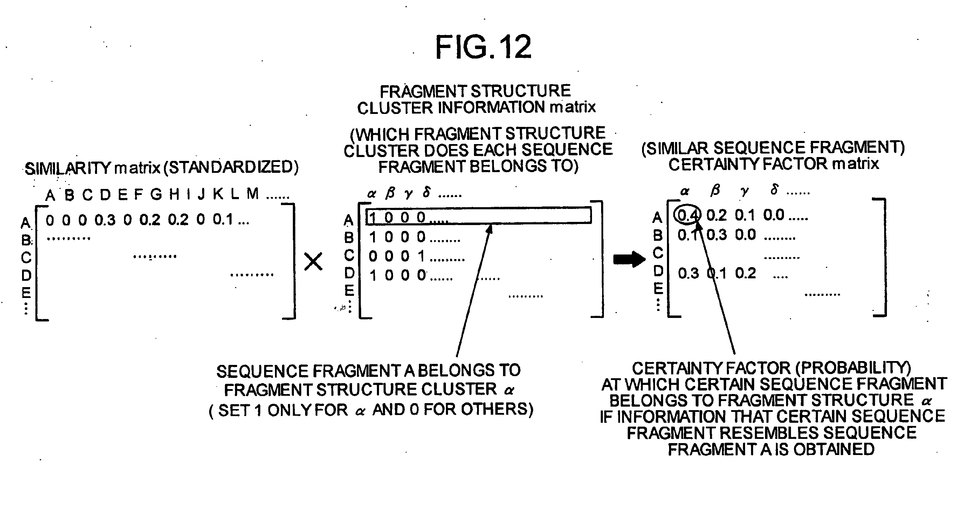

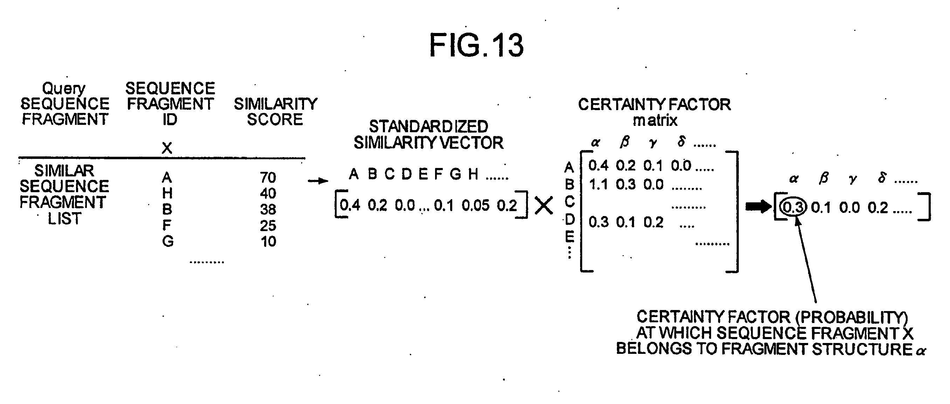

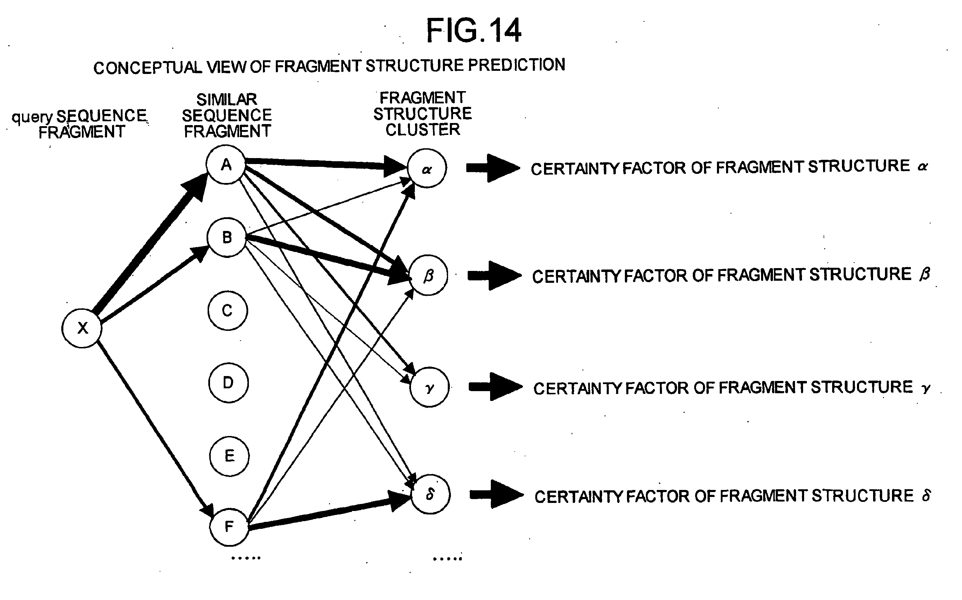

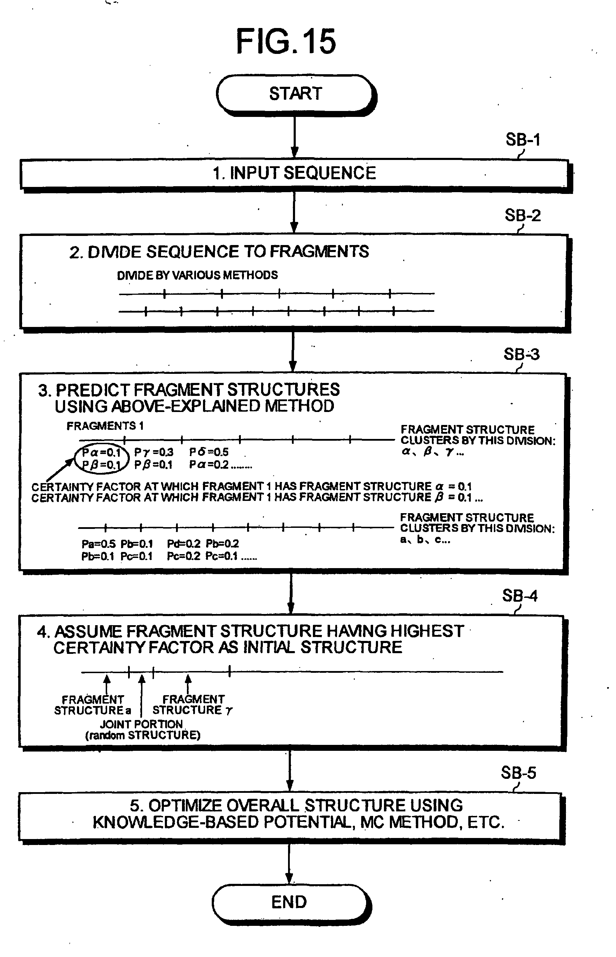

Fig. 1 illustrates one example in which a sequence is expressed by a profile according to the conventional art; Fig. 2 illustrates images of structure cluster creation according to the conventional art; Fig. 3 is a conceptual view which illustrates the basic principle of the present invention; Fig. 4 is a block diagram which illustrates one example of the configuration of a system to which the present invention is applied; Fig. 5 is a flow chart which illustrates one example of a fragment structure prediction processing performed by the system in one embodiment; Fig. 6 is a conceptual view which illustrates one example in which a fragment structure cluster creation section 102a obtains sequence fragments and corresponding fragment structures from a protein structure database 106a; Fig. 7 illustrates one example of fragment structure clusters of the sequence fragments created by the fragment structure creation section 102a; Fig. 8 illustrates one example of creating fragment structure clusters using a hierarchical clustering method; Fig. 9 is a conceptual view which illustrates an example of searching sequence fragments (D, F, G, S, I, and the like) resembling a sequence fragment A, similarity scores (50, 30, 28, 25, 20, and the like), of the sequence fragments, and fragment structure clusters ($\alpha$, $\alpha$, $\beta$, $\alpha$, $\gamma$, and the like) to which the respective sequence fragments belong; Fig. 10 illustrates one example of information stored in a similarity matrix 106b; Fig. 11 illustrates one example of information stored in a structure cluster information matrix 106c; Fig. 12 is a conceptual view which illustrates that a certainty factor matrix generation section 102e creates a certainty factor matrix 106d based on the similarity matrix 106b and the structure cluster information matrix 106c; Fig. 13 is a conceptual view which illustrates one example in which a similarity search is conducted to a query sequence (query sequence fragment) X, a search result is multiplied by the certainty factor matrix 106d, and a probability that the query sequence X belongs to the fragment structure; Fig. 14 is a conceptual view which illustrates one example of fragment structure prediction made by a sequence fragment structure prediction section 102j; and Fig. 15 is a flow chart which illustrates one example of an overall structure prediction processing performed by the system in this embodiment.

BEST MODE FOR CARRYING OUT THE INVENTION

Exemplary embodiments of a protein structure prediction device, a protein structure prediction method, a program, and a recording medium will be explained hereinafter in detail with reference to the drawings. It should be noted that this invention is not limited to the embodiments.

OUTLINE OF THE INVENTION

An outline of the present invention will be explained and then the configuration, processings, and the like of the present invention will be explained in detail. Fig. 3 is a conceptual view which illustrates the basic principle of the present invention.
Generally, the present invention has the following basic features. The present invention proposes a novel method of calculating a correlation between a local sequence and a local structure, with which method a complex correlation manifold can be expressed and the degree of the magnitude (certainty factor) of the correlation can be calculated.
According to the present invention, first, structure clusters of various magnitudes are created from various datasets and sequence similarity data is extracted from the structure clusters. After a user gives a query sequence, correlation clusters of structures to pseudo-dynamic sequences are created using the structure clusters of various magnitudes for divided, various local sequences and the magnitudes of the correlation clusters relative to the local sequences are calculated. Local structures are predicted from the correlation clusters.
A cluster creation process according to the present invention will be explained. According to the present invention, first, structures of sequence fragments are classified. Namely, based on sequence information and structure information stored in a known protein structure database or the like, typical fragment structures are extracted and classified.
As shown in Fig. 3(a), it is determined what structures sequence fragments present around a certain sequence fragment in a sequence space have. As shown in Fig. 3(b), it is determined what typical structures are obtained around the respective sequence fragments. It is thereby possible to create virtual clusters between sequences and structures. Namely, according to the present invention, it is determined which structure cluster each sequence present around a sequence A and resembling the sequence A belongs to in a structure space (which structure cluster each sequence belongs to when the sequence resembles the sequence A in what a way) by calculation, and a virtual cluster is created around this sequence. According to the present invention, when an unknown structure sequence fragment X is given, information on whether the fragment resembles the sequence A, or C and the like is acquired, virtual clusters are combined based on the information, and it is finally predicted which structure cluster the sequence belongs to.
The prediction of an overall structure is made by the following procedures according to the present invention. Degrees of magnitudes (certainty factors) of correlations are compared among obtained local structure candidates and the overall structure is predicted using the local structures having strong correlations and corresponding to long local sequences. The local structures (and those probabilities) having weak correlations are also held as data. Using these data, we can construct the other structures. We use these structures as next structure candidates through a folding simulation.

SYSTEM CONFIGURATION

A configuration of a system according to the present invention will first be explained. Fig. 4 is a block diagram which illustrates one example of the configuration of the system to which the present invention is applied. The system is generally constituted so that a protein structure prediction device 100 and an external system 200 which provides an external database about protein structure information and the like, an external program for homology search or the like, and the others are connected to each other so as to be able to hold communication between the systems 100 and 200 through a network 300.

In Fig. 4, the network 300 functions to connect the protein structure prediction device 100 to the external system 200 and may be, for example, the Internet.

In Fig. 4, the external system 200 is connected to the protein structure prediction device 100 through the network 300 and functions to provide the external database about the protein structure information and the like and a website on which the external analysis program for the homology search or the like is executed.

The external system 200 may be constituted as a WEB server, an ASP server or the like and the hardware configuration of the external system 200 may be such that the external system 200 consists of a commercially available information processing apparatus such as a workstation or a personal computer and peripherals of the apparatus. Respective functions of the external system 200 are realized by a central processing unit (hereinafter, "CPU"), a disk device, a memory device, an input device, an output device, a communication control device, and the like in the hardware configuration of the external system 200, a program that controls these constituent elements or the like.

In Fig. 4, the protein structure prediction device 100 generally consists of a control section 102, such as a CPU, which generally controls entirety of the protein structure prediction device 100, a communication control interface section 104 connected to a communication device (not shown), such as a router, connected to a communication line, an input and output control interface section 108 connected to an input device 112 and an output device 114, and a storage section 106 which stores various databases and tables (a protein structure database 106a to a certainty factor matrix 106d). The respective sections are connected to and communicable to one another through arbitrary communication paths. Further, this protein structure prediction device 100 is connected to and communicable to the network 300 through the communication device such as a router and a wired or wireless communication line such as a dedicated line.

The various databases and tables (the protein structure database 106a to the certainty factor matrix 106d) stored in the storage section 106, which are storage units such as fixed disk devices, store various programs, tables, databases, web page files, etc. used for various processings.

Among the constituent elements of the storage section 106, the protein structure database 106 is a database that stores protein structure information which records amino acid sequence information (primary structures) and three-dimensional structure information while making them correspond to one another. The protein structure database 106a is preferably a database from which sequence redundancy is eliminated. The protein structure database 106a may be an external protein structure database (for example, PDB_SELECT) accessed through the Internet or may be an in-house database created by copying these databases, storing original protein structures, and adding individual annotation information and the like.

A similarity matrix 106b is a matrix table that stores information on similarity search results about sequence fragments and the like.

A structure cluster information matrix 106c is a matrix table that stores information as to which fragment structure cluster each sequence fragment belongs to.

The certainty factor matrix 106d is a matrix table that stores information representing a certainty factor (a probability) at which a certain sequence fragment belongs to a fragment structure if information that the certain sequence fragment resembles the other sequence fragment is obtained.

In Fig. 4, the control section 102 includes an internal memory that stores control programs such as an operating system (hereinafter, "OS"), programs specifying various processing procedures, and required data. The control section 102 performs information processings for executing various processings. The control section 102 functionally conceptually consists of a fragment structure cluster creation section 102a, a sequence fragment similarity search section 102b, a similarity matrix creation section 102c, a structure cluster information matrix creation section 102d, a certainty factor matrix creation section 102e, a query sequence input section 102f, a query sequence fragment creation section 102g, a query sequence fragment similarity search section 102h, a fragment structure probability calculation section 102i, a sequence fragment structure prediction section 102j, and an overall structure optimization section 102k.

Among them, the fragment structure cluster creation section 102a is a fragment structure cluster creation unit that creates sequence fragments obtained by dividing sequence information to the sequence fragments each having a predetermined length and fragment structures corresponding to the sequence fragments based on the sequence information and the three-dimensional structure information on a protein, and that creates fragment structure clusters based on the similarities of the fragment structures. The sequence fragment similarity search section 102b is a sequence fragment similarity search unit that conducts sequence similarity searches for similarities between a sequence fragment and sequence fragments present around the sequence fragment in the sequence space. The similarity matrix creation section 102c is a similarity matrix creation unit that creates a similarity matrix representing results of the similarity searches about the sequence fragment conducted by the sequence fragment similarity search unit in the form of a sequence fragment matrix.

The structure cluster information matrix creation section 102d is a structure cluster information matrix creation unit that creates a structure cluster information matrix representing structure cluster information which indicates which fragment structure cluster each sequence fragment belongs in the form of a matrix of the sequence fragments and the structure clusters. The certainty factor matrix creation section 102e is a certainty factor matrix creation unit that creates a certainty factor matrix representing certainty factors which are probabilities that the sequences resembling a sequence fragment belong to the fragment structure clusters in the form of a matrix of the sequence fragments and the structure clusters.

The query sequence input section 102f is a query sequence input unit that allows a user to input a query sequence. The query sequence fragment creation section 102g is a query sequence fragment creation unit that creates query sequence fragments by dividing the query sequence input by the query sequence input unit to sequence fragments each having a predetermined length. The query sequence fragment similarity search section 102h is a query sequence fragment similarity search unit that conducts sequence similarity searches for similarities between the sequence fragment and the query sequence fragments created by the query sequence fragment creation unit. The fragment structure probability calculation section 102i is a fragment structure probability calculation unit that calculates a probability that each query sequence fragment belongs to the fragment structure cluster based on the search result of the query sequence fragment similarity search unit.

The sequence fragment structure prediction section 102j is a sequence fragment structure prediction unit that predicts a fragment structure of the query sequence based on the probability calculated by the fragment structure probability calculation unit. The overall structure optimization section 102k is an overall structure optimization unit that optimizes an initial overall structure determined by the fragment structure having the highest certainty factor in a predetermined manner. Details of processings performed by the respective sections will be explained later.

SYSTEM PROCESSINGS

One example of processings performed by the system thus constituted in this embodiment will be explained in detail with reference to Figs. 5 to 15.

FRAGMENT STRUCTURE PREDICTION PROCESSING

The detail of a fragment structure prediction processing will be explained with reference to Figs. 5 to 14. Fig. 5 is a flow chart which illustrates one example of the fragment structure prediction processing performed by the system in this embodiment.

The protein structure prediction device 100 accesses the protein structure database 106a, acquires the sequence information (for example, amino acid sequence information) and three-dimensional structure information on the protein, and creates sequence fragments obtained by dividing the sequence information to the sequence fragments each having a predetermined length and fragment structures corresponding to the respective sequence fragments by a processing performed by the fragment structure cluster creation section 102a (at step SA-1). Fig. 6 is a conceptual view which illustrates one example in which the fragment structure cluster creation section 102a acquires the sequence fragments and corresponding fragment structures from the protein structure database 106a. As shown in Fig. 6, the fragment structure cluster creation section 102a divides the sequence at intervals of a predetermined length (seven amino acid residues in Fig. 6) of a sequence fragment and stores the sequence fragments and fragment structures which the respective sequence fragments have in the storage section 106 while making the sequences and structures correspond to one another. The length of the fragment is not limited to the seven residues but can be one of various lengths.

The protein structure prediction device 100 then creates fragment structure clusters based on similarities of the fragment structures by a processing performed by the fragment structure cluster creation section 102a (at step SA-2). Fig. 7 illustrates one example of fragment structure clusters of the sequence fragments created by the fragment structure cluster creation section 102a. As shown in Fig. 7, the fragment structure cluster creation section 102a creates the clusters using a known clustering method such as a self organized map (SOM), a k-means, or a hierarchical clustering with the similarities of the fragment structures (for example, rmsd and dme) as similarity indexes.

Fig. 8 illustrates one example of creating the fragment structure clusters using the hierarchical clustering. As shown in Fig. 8, the fragment structure cluster creation section 102a calculates distances among all the fragment structures and sequentially collects the fragment structures having the shortest distances, thereby creating clusters. The distance between the clusters is calculated by, for example, calculating distances among all of the fragment structures belonging to each cluster and obtaining an average distance.

The protein structure prediction device 100 acquires similar sequence fragments, similarity scores, and fragment structure clusters which the respective sequence fragments belong to using a known sequence similarity search method such as a blast search method for similarities between all the sequence fragments and those present around the respective sequence fragments in the sequence space by a processing performed by the sequence fragment similarity search section 102b (at step SA-3). Fig. 9 is a conceptual view which illustrates an example of searching sequence fragments (D, F, G, S, I, and the like) resembling a sequence fragment A, similarity scores (50, 30, 28, 25, 20, and the like) of the sequence fragments, and fragment

structure clusters ($\alpha$, $\alpha$, $\beta$, $\alpha$, $\gamma$, and the like) to which the respective sequence fragments belong for the sequence fragment A.

The protein structure prediction device 100 creates the similarity matrix 106b representing results of similarity searches about the sequence fragments in the form of the matrix of the sequence fragments by a processing performed by the similarity matrix creation section 102c (at step SA-4). Fig. 10 illustrates one example of information stored in the similarity matrix 106b. As shown in Fig. 10, the similarity matrix 106b stores a result of executing similarity searches for the respective sequence fragments.

The protein structure prediction device 100 creates the structure cluster information matrix 106c representing which fragment structure cluster each sequence fragment belongs to by a processing performed by the structure cluster information matrix creation section 102d (at step SA-5). Fig. 11 illustrates one example of information stored in the structure cluster information matrix 106c. As shown in Fig. 11, structure cluster information "1" is set to the fragment structure cluster to which each sequence fragment belongs.

The protein structure prediction device 100 creates the certainty factor matrix 106d representing the certainty factor that is a probability that a certain sequence fragment belongs to the structure cluster of the other sequence fragment if information that the certain sequence fragment resembles the other sequence fragment is obtained by a processing performed by the certainty factor matrix creation section 102e (at step SA-6). Fig. 12 is a conceptual view which illustrates that the certainty factor matrix creation section 102e creates the certainty factor matrix 106d based on the similarity matrix 106b and the structure cluster information matrix 106c. As shown in Fig. 12, the certainty factor matrix creation section 102e creates the certainty factor matrix by calculating a product between the standardized similarity matrix 106b and the structure cluster information matrix 106c.

The protein structure prediction device 100 allows the user to input the query sequence by a processing performed by the query sequence input section 102f (at step SA-7). This sequence may be input by allowing the user to select a desired sequence from an external database that stores amino acid sequences or by allowing the user to directly input the desired sequence.

The protein structure prediction device 100 divides the query sequence at intervals of the predetermined length (for example, seven amino acid residues) of a sequence fragment and stores the sequence fragments (query sequence fragments) in the storage section 106 by a processing performed by the query sequence fragment creation section 102g (atstep SA-8). The length of the fragment is not limited to the seven amino acid residues but may be one of various lengths.

The protein structure prediction device 100 searches sequence similarities of the respective sequence fragments of the query sequences (query sequence fragments) by a processing performed by the query sequence fragment similarity search section 102h (at step SA-9) and calculates probabilities of the fragment structures to which the respective sequence fragments belong by a processing performed by the fragment structure probability calculation section 102i based on the search result (at step SA-10). Fig. 13 is a conceptual view which illustrates one example in which a similarity search is conducted for a query sequence (query sequence fragment) X, the search result is multiplied by the certainty factor matrix 106d, and the probability that the query sequence X belongs to each fragment structure is thereby calculated. As shown in Fig. 13, by multiplying a standardized similarity vector of the query sequence X, the probability (certainty factor) at which the query sequence X belongs to each fragment structure cluster can be calculated.

The protein structure prediction device 100 predicts the fragment structure of the query sequence based on these calculated probabilities (certainty factors) by a processing performed by the sequence fragment structure prediction section 102j (at step SA-11). Fig. 14 is a conceptual view which illustrates one example of the fragment structure prediction made by the sequence fragment structure prediction section 102j. As shown in Fig. 14, the sequence fragment structure prediction section 102j sorts the structure clusters to which the respective similar sequences to the query sequence X belong according to their certainty factors, thereby predicting that the query sequence X belongs to the fragment structure $\alpha$. The fragment structure prediction processing is thus finished.

OVERALL STRUCTURE PREDICTION PROCESSING

The detail of the overall structure prediction processing will next be explained with reference to Fig. 15. Fig. 15 is a flow chart which illustrates one example of the overall structure prediction processing performed by the system in this embodiment.

The user inputs the query sequence first (at step SB-1).

The protein structure prediction device 100 divides the query sequence at intervals of the predetermined length of a sequence fragment by a processing performed by the query sequence fragment creation section 102g (at step SB-2). At the step, the device 100 creates a plurality of patterns of divided sequence fragments by different lengths (two patterns in Fig. 15).

The protein structure prediction device 100 predicts the fragment structures using the above-explained method (at step SB-3).

The protein structure prediction device 100 creates an initial overall structure based on the fragment structure having the highest certainty factor by a processing performed by the sequence fragment structure prediction section 102j (at step SB-4).

The protein structure prediction device 100 conducts optimization for the overall structure with reference to the overall structure optimization section 102k, using a knowledge-based potential method, an MC method, a simulated annealing (SA) method or the like (at step SB-5).

One example of the optimization will be explained as follows.

(1) Calculate an energy ($E_{old}$) of the overall structure;

(2) For a joint portion, move a dihedral angle at random, calculate an energy ($E_{new}$) after moving the dihedral angle, and calculate a probability that the moved dihedral angle is adopted at the next step as expressed by:

$$\rho = \exp(-\beta\Delta E) \text{ (where } \Delta E = E_{new} - E_{old});$$

and

(3) For the fragment structures, substitute the structures by randomly selecting one of predicted fragment structures, calculate the energy ($E_{new}$) at a certainty factor ($P_{new}$) after substitution, and calculate the probability $\rho$ that the substituting fragment structure is adopted at the next step as expressed by:

$$\rho = P_{new} \exp(-\beta E_{new})/P_{old} \exp(-\beta E_{old}).$$

By repeating (1) to (3), the optimization is conducted. The overall structure prediction processing is thus finished.

OTHER EMBODIMENTS

An embodiment of the present invention has been explained so far. However, the present invention may be carried out in various other embodiments within the scope of a technical concept defined in the appended claims.

For example, the example in which the protein structure prediction device 100 performs processings in a standalone fashion has been explained. However, the system may be constituted so that the processings are carried out in accordance with requests from a client terminal constituted separately from the protein structure prediction device 100 and so that processing results are returned to the client terminal.

Further, among the processings explained in the embodiment, all of or part of those explained to be performed automatically can be performed manually or all of or part of those explained to be performed manually can be performed automatically.

The processing procedures, the control procedures, the specific names, the information including various pieces of registered data and parameters such as search conditions, the examples of screens, and the database configurations explained or shown in the drawings can be arbitrarily changed unless specified otherwise.

The respective constituent elements of the protein structure prediction device 100 shown in the drawings are functionally conceptual and the device 100 is not always constituted physically as shown in the drawings.

For example, all of or arbitrary part of the processing functions of the respective sections (respective devices) of the protein structure prediction device 100, particularly the respective processing functions executed by the control section, can be realized by the CPU and a program interpreted and executed by the CPU or can be realized as wired logic hardware. The program is recorded on a recording medium to be explained later and mechanically read by the protein structure prediction device 100 at need. That is, a computer program for transmitting a command to the CPU in cooperation with the OS and performing the various processings is recorded on the storage section 106 or the like such as a read only memory (hereinafter, "ROM") or a hard disk (hereinafter, "HD"). This computer program is executed by being loaded to a random access memory (hereinafter, "RAM") and the computer program and the CPU constitute the control section.

However, this computer program may be recorded on an application program server connected to the protein structure prediction device 100 through an arbitrary network and all of or part of the computer program can be downloaded at need.

The program according to the present invention can be stored in a computer readable recording medium. Examples of this "recording medium" include arbitrary "portable physical mediums" such as a flexible disk, a magneto optical disk, the ROM, an erasable programmable ROM (EPROM), an electric EPROM (EERROM), a CD-ROM, a magneto optical (MO), and a digital video disk (hereinafter, "DVD"), arbitrary "fixed physical mediums" such as the ROM, the RAM, and the HD included in various types of computer systems, and "communication mediums" for holding the program for a short period of time such as a communication line and a carrier wave used when the program is transmitted through a network represented by a local area network (hereinafter, "LAN"), a wide area network (WAN), and the Internet.

Furthermore, the "program" is a data processing method described in an arbitrary language or by an arbitrary description method. The form of the program is not limited to a specific one but may be a source code or a binary code. It is noted that the "program" is not always limited to the program constituted unitarily but examples thereof include a program constituted to be distributed as a plurality of modules or libraries and a program for attaining functions of the program in cooperation with a different program represented by the OS. For the specific configurations, reading procedures, installation procedures after reading, and the like for reading the recording medium by the respective devices explained in the embodiment, well-known configurations and procedures can be used.

The network 300 functions to connect the protein structure prediction device 100 to the external system 200 and may include one of, for example, the Internet, an intranet, the LAN (which may be either wired or wireless), a value added network (VAN), a personal computer communication network, a public telephone network (which may be either analog or digital), a dedicated network (which may be either analog or digital), a cable television (CATV) network, a portable line network/portable packet switching network according to International Mobile Telecommunications (IMT) 2000, Global Systems for Mobile Communications (GSM) or PDC/PDC-P, a wireless call network, a local wireless network such as one according to Bluetooth, a personal handy phone system (PHS) network, and satellite communication networks such as communication satellite (CS), broadcasting satellite (BS), and integrated services digital broadcasting (ISDB) networks. Namely, the system according to the present invention can transmit and receive various pieces of data through the arbitrary network which may be either wired or wireless.

The various databases and the like (the protein structure database 106a to the certainty factor matrix 106d) stored in the storage section 106 are storage units such as memory devices, for example, the RAM and the ROM, fixed disk devices such as the hard disks, the flexible disks, the optical disks, and the like. The databases and the like store various programs, tables, files, databases, web page files, and the like used for the various processings and for providing a website.

The protein structure prediction device 100 may be realized by connecting peripherals such as a printer, a monitor, and an image scanner to an information processing apparatus such as an information processing terminal, for example, a known personal computer or workstation and by installing software (including programs, data, and the like) for allowing the information processing apparatus to realize the method of the present invention.

Moreover, the specific manners of the distribution or integration of the protein structure prediction device 100 are not limited to those shown in the drawings. All of or part of the constituent elements of the device 100 can be constituted by functionally or physically distributing or integrating them in arbitrary units according to various loads and the like. For example, the respective databases may be constituted individually as independent database devices or all of or part of the processings may be realized using a common gateway interface (CGI).

As explained so far in detail, according to the present invention, sequence fragments obtained by dividing sequence information at intervals of a predetermined length and fragment structures corresponding to the sequence fragments are created based on the sequence information and three-dimensional structure information on a protein, fragment structure clusters are created based on similarities of the fragment structures, sequence similarity searches are conducted for similarities between the sequence fragments and the sequence fragments present around the sequence fragments in a sequence space, respectively, and a certainty factor matrix representing a certainty factor that is a probability that a sequence resembling each of the sequence fragments belongs to each of the fragment structure clusters in the form of a matrix of the sequence fragments and the structure clusters is created. A user is allowed to input a query matrix, the input query sequence is divided at intervals of a predetermined length to thereby create query sequence fragments, sequence similarity searches are conducted for similarities between the created query sequence fragments and the sequence fragment, respectively, the probability that each of the query sequence fragments belongs to each of the fragment structure clusters is calculated based on the created certainty factor matrix and search results, and the fragment structure of the query sequence is predicted based on the calculated probability. Therefore, it is possible to calculate a correlation of a local structure from a local sequence so as to be able to express a complex manifold of the correlation and predict the partial structure. Namely, the present invention can provide the protein structure prediction device, the protein structure prediction method, the program, and the recording medium capable of giving and holding probabilities (certainty factors) of a plurality of structure candidates according to their degrees of correlations when calculating the structure (using a certainty factor function as a probability of structure change).

Further, the technique that the protein structure is assumed as a block of local structures each having a high correlation is conventionally known. However, the present invention can provide the protein structure prediction device, the protein structure prediction method, the program, and the recording medium which enable the device of the present invention to create clusters of local structures first, consider a complex form of a structure sequence correlation manifold, and dynamically create sequence correlation clusters after the query sequence is given.

In addition, the present invention can provide the protein structure prediction device, the protein structure prediction method, the program, and the recording medium capable of creating many structure clusters from different viewpoints (for example, lengths of sequence fragments, resolutions of fragment structures, magnitudes of structure clusters, and degrees of correlations) and calculating the structure by integrating structure prediction results from the respective datasets.

Further, according to the present invention, a similarity matrix creation unit which creates a similarity matrix representing the results of the similarity searches conducted by the sequence fragment similarity search unit for the sequence fragments in the form of a matrix of the sequence fragments is provided, a cluster information matrix representing structure cluster information that indicates which of the fragment structure clusters each of the sequence fragments belongs to in the form of a matrix of the sequence fragments and the structure clusters is created, and the certainty factor matrix is created based on the similarity matrix and the structure cluster information matrix thus created. Therefore, the present invention can provide the protein structure prediction device, the protein structure prediction method, the program, and the recording medium capable of easily, finely calculating certainty factors based on the similarity search results using a matrix operation technique.

Moreover, according to the present invention, predetermined optimization is conducted to an initial overall structure determined by the fragment structure having the highest certainty factor. Therefore, the sequence can be divided to various possible sequence fragments and their optimum prediction results can be integrated when creating the initial structure. In addition, the present invention can provide the protein structure prediction device, the protein structure prediction method, the program, and the recording medium capable of further improving accuracy for the overall structure prediction by further optimizing the initial structure.

INDUSTRIAL APPLICABILITY

As explained so far, the protein structure prediction device, the protein structure prediction method, the program, and the recording medium according to the present invention can be employed for the development of new drugs and the like using the prediction of the protein structure, the analysis of mutual regions of the protein, and the analysis results.

**Claims**

1. A protein structure prediction device comprising:

   a fragment structure cluster creation unit that based on sequence information and three-dimensional structure information on a protein, creates a plurality of sequence fragments obtained by dividing the sequence information at intervals of a predetermined length and a plurality of fragment structures corresponding to the respective sequence fragments, and that creates a plurality of fragment structure clusters based on similarities of the fragment structures;
   a sequence fragment similarity search unit that performs sequence similarity search for similarities between the sequence fragments and the sequence fragments present around the sequence fragments in a sequence space, respectively;
   a certainty factor matrix creation unit that creates a certainty factor matrix which represents a certainty factor in a form of a matrix of the sequence fragments and the structure clusters, the certainty factor being a probability that the similar sequence belongs to a fragment structure cluster out of the fragment structure clusters;
   a query sequence input unit that allows a user to input a query sequence;
   a query sequence fragment creation unit that divides the query sequence input by the query sequence input unit at intervals of a predetermined length to create query sequence fragments;
   a query sequence fragment similarity search unit that performs sequence similarity search for similarities between each of the query sequence fragments created by the query sequence fragment creation unit and each of the sequence fragments;
   a fragment structure probability calculation unit that calculates a probability that each of the query sequence fragments belongs to each of the fragment structure clusters based on the certainty factor matrix created by the certainty factor matrix creation unit and a search result of the query sequence fragment similarity search unit; and
   a sequence fragment structure prediction unit that predicts a fragment structure of the query sequence based on the probability calculated by the fragment structure probability calculation unit.

2. The protein structure prediction device according to claim 1, further comprising:

   a similarity matrix creation unit that creates a similarity matrix which represents a search result of the sequence fragment similarity search unit in a form of a matrix of the sequence fragments; and
   a structure cluster information matrix creation unit that creates a structure cluster information matrix which represents structure cluster information in a form of a matrix of the sequence fragments and the structure clusters, the structure cluster information indicating which of the fragment structure clusters each of the sequence fragments belongs to, wherein
   the certainty factor matrix creation unit creates the certaintyfactor matrix based on the similarity matrix created by the similarity matrix creation unit and the structure cluster information matrix.

3. The protein structure prediction device according to claim 1 or 2, further comprising:

an overall structure optimization unit that performs predetermined optimization to an initial overall structure determined by a fragment structure, out of the fragment structures, having a highest certainty factor.

4. A protein structure prediction method comprising:

a fragment structure cluster creation step of creating, based on sequence information and three-dimensional structure information on a protein, a plurality of sequence fragments obtained by dividing the sequence information at intervals of a predetermined length and a plurality of fragment structures corresponding to the respective sequence fragments, and of creating a plurality of fragment structure clusters based on similarities of the fragment structures;
a sequence fragment similarity search step of performing sequence similarity search for similarities between the sequence fragments and the sequence fragments present around the sequence fragments in a sequence space, respectively;
a certainty factor matrix creation step of creating a certainty factor matrix which represents a certainty factor in a form of a matrix of the sequence fragments and the structure clusters, the certainty factor being a probability that the similar sequence belongs to a fragment structure cluster out of the fragment structure clusters;
a query sequence input step of allowing a user to input a query sequence;
a query sequence fragment creation step of dividing the query sequence at intervals of a predetermined length to create query sequence fragments;
a query sequence fragment similarity search step of performing sequence similarity search for similarities between each of the query sequence fragments created by the query sequence fragment creation step and each of the sequence fragments;
a fragment structure probability calculation step of calculating a probability that each of the query sequence fragments belongs to each of the fragment structure clusters based on the certainty factor matrix created by the certainty factor matrix creation step and a search result of the performing sequence similarity search;

and
a sequence fragment structure prediction step of predicting a fragment structure of the query sequence based on the probability calculated by the fragment structure probability calculation step.

5. A protein structure prediction method according to claim 1, further comprising:

a similarity matrix creation step of creating a similarity matrix which represents a search result of the sequence fragment similarity search unit in a form of a matrix of the sequence fragments; and
a structure cluster information matrix creation step of creating a structure cluster information matrix which represents structure cluster information in a form of a matrix of the sequence fragments and the structure clusters, the structure cluster information indicating which of the fragment structure clusters each of the sequence fragments belongs to, wherein
the certainty factor matrix creation step includes creating the certainty factor matrix based on the similarity matrix created by the similarity matrix creation step and the structure cluster information matrix created by the similarity matrix creation step.

6. The protein structure prediction method according to claim 1 or 2, further comprising:

an overall structure optimization step of performing predetermined optimization to an initial overall structure determined by a fragment structure, out of the fragment structures, having a highest certainty factor.

7. A program which allows a computer to execute a protein structure prediction method comprising:

a fragment structure cluster creation step of creating, based on sequence information and three-dimensional structure information on a protein, a plurality of sequence fragments obtained by dividing the sequence information at intervals of a predetermined length and a plurality of fragment structures corresponding to the respective sequence fragments, creating a plurality of fragment structure clusters based on similarities of the fragment structures;
a sequence fragment similarity search step of performing sequence similarity search for similarities between the sequence fragments and the sequence fragments present around the sequence fragments in a sequence space, respectively;

a certainty factor matrix creation step of creating a certainty factor matrix which represents a certainty factor in a form of a matrix of the sequence fragments and the structure clusters, the certainty factor being a probability that the similar sequence belongs to a fragment structure cluster out of the fragment structure clusters;

a query sequence input step of allowing a user to input a query sequence;

a query sequence fragment creation step of dividing the query sequence at intervals of a predetermined length to create query sequence fragments;

a query sequence fragment similarity search step of performing sequence similarity search for similarities between each of the query sequence fragments created by the query sequence fragment creation step and each of the sequence fragments;

a fragment structure probability calculation step of calculating a probability that each of the query sequence fragments belongs to each of the fragment structure clusters based on the certainty factor matrix created by the certainty factor matrix creation step and a search result of the performing sequence similarity search; and

a sequence fragment structure prediction step of predicting a fragment structure of the query sequence based on the probability calculated by the fragment structure probability calculation step.

8. The program according to claim 7, wherein the protein structure prediction method further comprising:

a similarity matrix creation step of creating a similarity matrix which represents a search result of the sequence fragment similarity search unit in a form of a matrix of the sequence fragments; and

a structure cluster information matrix creation step of creating a structure cluster information matrix which represents structure cluster information in a form of a matrix of the sequence fragments and the structure clusters, the structure cluster information indicating which of the fragment structure clusters each of the sequence fragments belongs to, wherein

the certainty factor matrix creation step includes creating the certainty factor matrix based on the similarity matrix created by the similarity matrix creation step and the structure cluster information matrix created by the similarity matrix creation step.

9. The program according to claim 7 or 8, wherein the protein structure prediction method further comprising:

an overall structure optimization step of performing predetermined optimization to an initial overall structure determined by a fragment structure, out of the fragment structures, having a highest certainty factor.

10. A computer readable recording medium which records a program which allows a computer to execute a protein structure prediction method comprising:

a fragment structure cluster creation step of creating, based on sequence information and three-dimensional structure information on a protein, a plurality of sequence fragments obtained by dividing the sequence information at intervals of a predetermined length and a plurality of fragment structures corresponding to the respective sequence fragments, and of creating a plurality of fragment structure clusters based on similarities of the fragment structures;

a sequence fragment similarity search step of performing sequence similarity search for similarities between the sequence fragments that are located near each other the sequence fragments and the sequence fragments present around the sequence fragments in a sequence space, respectively;

a certainty factor matrix creation step of creating a certainty factor matrix which represents a certainty factor in a form of a matrix of the sequence fragments and the structure clusters, the certainty factor being a probability that the similar sequence belongs to a fragment structure cluster out of the fragment structure clusters;

a query sequence input step of allowing a user to input a query sequence;

a query sequence fragment creation step of dividing the query sequence at intervals of a predetermined length to create query sequence fragments;

a query sequence fragment similarity search step of performing sequence similarity search for similarities between each of the query sequence fragments created by the query sequence fragment creation step and each of the sequence fragments;

a fragment structure probability calculation step of calculating a probability that each of the query sequence fragments belongs to each of the fragment structure clusters based on the certainty factor matrix created by the certainty factor matrix creation step and a search result of the performing sequence similarity search;

and
a sequence fragment structure prediction step of predicting a fragment structure of the query sequence based on the probability calculated by the fragment structure probability calculation step.

**11.** The computer readable recording medium according to claim 10, wherein the protein structure prediction method further comprising:

a similarity matrix creation step of creating a similarity matrix which represents a search result of the sequence fragment similarity search unit in a form of a matrix of the sequence fragments; and
a structure cluster information matrix creation step of creating a structure cluster information matrix which represents structure cluster information in a form of a matrix of the sequence fragments and the structure clusters, the structure cluster information indicating which of the fragment structure clusters each of the sequence fragments belongs to, wherein
the certainty factor matrix creation step includes creating the certainty factor matrix based on the similarity matrix created by the similarity matrix creation step and the structure cluster information matrix created by the similarity matrix creation step.

**12.** The computer readable recording medium according to claim 10 or 11, wherein the protein structure prediction method further comprising:

an overall structure optimization step of performing predetermined optimization to an initial overall structure determined by a fragment structure, out of the fragment structures, having a highest certainty factor.

# FIG.1

**(a)**

```
E.G.) SEQUENCE profile IF
      SEQUENCE FRAGMENT IS AGGED

    A C D E F G H I K L M N P Q R S T V W Y
1   1 0 0 0 0 0 0 0 0 0 0 0 0 0 0 0 0 0 0 0
2   0 0 0 0 0 1 0 0 0 0 0 0 0 0 0 0 0 0 0 0
3   0 0 0 0 0 1 0 0 0 0 0 0 0 0 0 0 0 0 0 0
4   0 0 0 1 0 0 0 0 0 0 0 0 0 0 0 0 0 0 0 0
5   0 0 1 0 0 0 0 0 0 0 0 0 0 0 0 0 0 0 0 0
```

**(b)**

```
E.G.) CLUSTER SEQUENCE profile IF
      SEQUENCE FRAGMENTS AGGED
      AND ADGDD CONSTITUTE CLUSTER

    A C D   E  F G  H I K K M N P Q R S T V W Y
1   1 0 0   0  0 0  0 0 0 0 0 0 0 0 0 0 0 0 0 0
2   1 0 0.5 0  0 0.50 0 0 0 0 0 0 0 0 0 0 0 0 0
3   0 0 0   0  0 1  0 0 0 0 0 0 0 0 0 0 0 0 0 0
4   0 0 0.5 0.50 0  0 0 0 0 0 0 0 0 0 0 0 0 0 0
5   0 0 1   0  0 0  0 0 0 0 0 0 0 0 0 0 0 0 0 0
```

# FIG.2

IMAGES OF CLUSTER CREATION ACCORDING TO CONVENTIONAL ART

(a)

SEQUENCE FRAGMENT SPACE

(b)

POINT IN CORRESPONDING
FRAGMENT STRUCTURE SPACE

SEQUENCE
FRAGMENT SPACE

FRAGMENT
STRUCTURE SPACE

(c)

EXPRESSION OF
ONE CREATED
CLUSTER IN SEQUENCE
FRAGMENT SPACE

EXPRESSION OF
ONE CREATED
CLUSTER IN FRAGMENT
STRUCTURE SPACE

SEQUENCE
FRAGMENT SPACE

FRAGMENT
STRUCTURE SPACE

EP 1 460 559 A1

# FIG.3

EP 1 460 559 A1

(BASIC PRINCIPLE OF THE PRESENT INVENTION)

(a)

CERTAIN SEQUENCE FRAGMENT A

FRAGMENT STRUCTURE CLUSTER α

FRAGMENT STRUCTURE CLUSTER β

SEQUENCE FRAGMENT SPACE

FRAGMENT STRUCTURE SPACE

SET OF SEQUENCE FRAGMENTS PRESENT AROUND CERTAIN SEQUENCE FRAGMENT A

FRAGMENT STRUCTURE CLUSTER γ

(b)

FRAGMENT STRUCTURE CLUSTER α REGION

CERTAIN SEQUENCE FRAGMENT A

FRAGMENT STRUCTURE CLUSTER β REGION

FRAGMENT STRUCTURE CLUSTER γ REGION

SEQUENCE FRAGMENT SPACE

**FIG.4**

EP 1 460 559 A1

# FIG.5

(<u>FRAGMENT STRUCTURE PREDICTION PROCESSING</u>)

START

OBTAIN SEQUENCE FRAGMENTS AND FRAGMENT STRUCTURES OF SEQUENCE FRAGMENTS FROM PROTEIN STRUCTURE DB — SA-1

CREATE FRAGMENT STRUCTURE CLUSTERS BASED ON SIMILARITIES OF FRAGMENT STRUCTURES — SA-2

SEARCH SEQUENCE SIMILARITIES FOR ALL SEQUENCE FRAGMENTS — SA-3

CREATE SIMILARITY matrix — SA-4

CREATE STRUCTURE CLUSTER INFORMATION matrix — SA-5

CREATE CERTAINTY FACTOR matrix REPRESENTING CERTAINTY FACTOR THAT IS PROBABILITY CERTAIN SEQUENCEFRAGMENT BELONGS TO STRUCTURE CLUSTER OF THE OTHER SEQUENCE FRAGMENT — SA-6

INPUT query SEQUENCE — SA-7

CREATE SEQUENCE FRAGMENTS FOR query SEQUENCE — SA-8

SEARCH SEQUENCE SIMILARITIES OF SEQUENCE FRAGMENTS OF query SEQUENCE — SA-9

CALCULATE PROBABILITY OF FRAGMENT STRUCTURE TO WHICH SEQUENSE FRAGMENT BELONG — SA-10

PREDICT FRAGMENT STRUCTURE OF SEQUENCE FRAGMENT — SA-11

END

# FIG.6

160a
PROTEIN STRUCTURE
DATABASE

PROTEIN A  PROTEIN B  PROTEIN C  PROTEIN D  PROTEIN E  o o o

EXTRACT SEQUENCE FRAGMENTS USING ALL INFORMATION IN DATABASE
(E.G., LENGTH OF SEVEN RESIDUES)

| SEQUENCE FRAGMENT ID | SEQUENCE FRAGMENT | FRAGMENT STRUCTURE |
|---|---|---|
| A | MMLQGLE | |
| B | MLQGLES | |
| C | LQGLESP | |
| D | QGLESPS | |
| E | GLWSPSF | |

EP 1 460 559 A1

FIG.7

# FIG.8

# FIG.9

| | SEQUENCE FRAGMENT ID | SEQUENCE FRAGMENT | SIMILARITY SCORE | FRAGMENT STRUCTURE ID |
|---|---|---|---|---|
| SEQUENCE FRAGMENT | A | MMLQGLE | — | α |
| SIMILAR SEQUENCE FRAGMENT LIST | D | MMIEGLD | 50 | α |
| | F | MDLQGDD | 30 | α |
| | G | DMLGGLD | 28 | β |
| | S | MDIDGID | 25 | α |
| | I | MDIGGII | 20 | γ |
| | .......... | | | |

EP 1 460 559 A1

# FIG.10

SIMILARITY matrix

A B C D E F G H I J K L M N O P Q R .......

A  0 0 0 50 0 30 28 0 20 ....
B  .......
C       .........
D
E                .........
·
·
·

STORE RESULTS
OF SIMILARITY
SEARCHES ABOUT
SEQUENCE
FRAGMENT A

26

# FIG.11

STRUCTURE CLUSTER INFORMATION matrix
(WHICH FRAGMENT STRUCTURE CLUSTER DOES
EACH SEQUENCE FRAGMENT BELONGS TO)

$$\alpha \quad \beta \quad \gamma \quad \delta \quad ....... $$

|   | | | | |
|---|---|---|---|---|
| A | 1 | 0 | 0 | 0 ..... |
| B | 1 | 0 | 0 | 0 ....... |
| C | 0 | 0 | 0 | 1 ......... |
| D | 1 | 0 | 0 | 0 ..... .... |
| E | | | | ... .... |

SEQUENCE FRAGMENT A BELONGS TO
FRAGMENT STRUCTURE CLUSTER $\alpha$
( SET 1 ONLY FOR $\alpha$ AND 0 FOR OTHERS)

# FIG.12

FRAGMENT STRUCTURE
CLUSTER INFORMATION matrix

(WHICH FRAGMENT STRUCTURE
CLUSTER DOES EACH SEQUENCE
FRAGMENT BELONGS TO)

(SIMILAR SEQUENCE FRAGMENT)
CERTAINTY FACTOR matrix

SIMILARITY matrix (STANDARDIZED)

A B C D E F G H I J K L M ......

$\alpha$ $\beta$ $\gamma$ $\delta$ ......

$\alpha$ $\beta$ $\gamma$ $\delta$ ......

$$
\begin{array}{c} A \\ B \\ C \\ D \\ E \\ \vdots \end{array}
\begin{bmatrix} 0 & 0 & 0 & 0.3 & 0 & 0.2 & 0.2 & 0 & 0.1 \ldots \\ \ldots \ldots \\ \ldots \ldots \\ \ldots \ldots \\ \\ \end{bmatrix}
\times
\begin{array}{c} A \\ B \\ C \\ D \\ E \\ \vdots \end{array}
\begin{bmatrix} 1 & 0 & 0 & 0 \ldots \\ 1 & 0 & 0 & 0 \ldots \\ 0 & 0 & 0 & 1 \ldots \\ 1 & 0 & 0 & 0 \ldots \\ \ldots \\ \end{bmatrix}
\Rightarrow
\begin{array}{c} A \\ B \\ C \\ D \\ E \\ \vdots \end{array}
\begin{bmatrix} 0.4 & 0.2 & 0.1 & 0.0 \ldots \\ 0.1 & 0.3 & 0.0 \ldots \\ \ldots \\ 0.3 & 0.1 & 0.2 & \ldots \\ \ldots \\ \end{bmatrix}
$$

SEQUENCE FRAGMENT A BELONGS TO
FRAGMENT STRUCTURE CLUSTER $\alpha$
( SET 1 ONLY FOR $\alpha$ AND 0 FOR OTHERS)

CERTAINTY FACTOR (PROBABILITY)
AT WHICH CERTAIN SEQUENCE FRAGMENT
BELONGS TO FRAGMENT STRUCTURE $\alpha$
IF INFORMATION THAT CERTAIN SEQUENCE
FRAGMENT RESEMBLES SEQUENCE
FRAGMENT A IS OBTAINED

# FIG.13

CERTAINTY FACTOR (PROBABILITY) AT WHICH SEQUENCE FRAGMENT X BELONGS TO FRAGMENT STRUCTURE $\alpha$

EP 1 460 559 A1

# FIG.14

CONCEPTUAL VIEW OF FRAGMENT STRUCTURE PREDICTION

query SEQUENCE FRAGMENT

SIMILAR SEQUENCE FRAGMENT

FRAGMENT STRUCTURE CLUSTER

CERTAINTY FACTOR OF FRAGMENT STRUCTURE $\alpha$

CERTAINTY FACTOR OF FRAGMENT STRUCTURE $\beta$

CERTAINTY FACTOR OF FRAGMENT STRUCTURE $\gamma$

CERTAINTY FACTOR OF FRAGMENT STRUCTURE $\delta$

EP 1 460 559 A1

# FIG.15

START

SB-1

1. INPUT SEQUENCE

SB-2

2. DIVIDE SEQUENCE TO FRAGMENTS

DIVIDE BY VARIOUS METHODS

SB-3

3. PREDICT FRAGMENT STRUCTURES
USING ABOVE-EXPLAINED METHOD

FRAGMENTS 1

$P\alpha=0.1$ $P\gamma=0.3$ $P\delta=0.5$
$P\beta=0.1$ $P\beta=0.1$ $P\alpha=0.2$ ........

FRAGMENT STRUCTURE
CLUSTERS BY THIS DIVISION:
$\alpha$、 $\beta$、 $\gamma$ ...

CERTAINTY FACTOR AT WHICH FRAGMENT 1 HAS FRAGMENT STRUCTURE $\alpha$ = 0.1
CERTAINTY FACTOR AT WHICH FRAGMENT 1 HAS FRAGMENT STRUCTURE $\beta$ = 0.1 ...

FRAGMENT STRUCTURE
CLUSTERS BY THIS DIVISION:
a、 b、 c...

Pa=0.5 Pb=0.1 Pd=0.2 Pb=0.2
Pb=0.1 Pc=0.1 Pc=0.2 Pc=0.1 ......

SB-4

4. ASSUME FRAGMENT STRUCTURE HAVING HIGHEST
CERTAINTY FACTOR AS INITIAL STRUCTURE

FRAGMENT
STRUCTURE a

FRAGMENT
STRUCTURE $\gamma$

JOINT PORTION
(random STRUCTURE)

SB-5

5. OPTIMIZE OVERALL STRUCTURE USING
KNOWLEDGE-BASED POTENTIAL, MC METHOD, ETC.

END

## INTERNATIONAL SEARCH REPORT

| International application No. |
| --- |
| PCT/JP02/13832 |

| A. CLASSIFICATION OF SUBJECT MATTER |
| --- |
| Int.Cl⁷ G06F17/30, G06F19/00, C07K1/00 |

According to International Patent Classification (IPC) or to both national classification and IPC

| B. FIELDS SEARCHED |
| --- |

Minimum documentation searched (classification system followed by classification symbols)
Int.Cl⁷ G06F17/30, G06F19/00, C07K1/00

Documentation searched other than minimum documentation to the extent that such documents are included in the fields searched

| | | | |
| --- | --- | --- | --- |
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)
JICST FILE(JOIS), WPI, INSPEC(DIALOG)

| C. DOCUMENTS CONSIDERED TO BE RELEVANT |
| --- |

| Category* | Citation of document, with indication, where appropriate, of the relevant passages | Relevant to claim No. |
| --- | --- | --- |
| A | YI, T-M. et al., Protein Secondary Structure Prediction Using Nearest-neighbor Methods. JOURNAL OF MOLECULAR BIOLOGY, 1993, Vol.232, No.4, pages 1117 to 1129 | 1-12 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 27 February, 2003 (27.02.03) | 11 March, 2003 (11.03.03) |

| Name and mailing address of the ISA/ | Authorized officer |
| --- | --- |
| Japanese Patent Office | |
| Facsimile No. | Telephone No. |

Form PCT/ISA/210 (second sheet) (July 1998)

32